# EUROPEAN PATENT SPECIFICATION

(11) **EP 4 095 864 B1**
(45) Date of publication and mention of the grant of the patent: **18.11.2026**
(21) Application number: 22169697.4
(22) Date of filing: 30.05.2012
(51) Int. Cl.: G16H 10/60, G16H 40/67

(54) **METHOD AND SYSTEM FOR ACQUIRING PATIENT-RELATED DATA**
VERFAHREN UND SYSTEM FÜR DEN ZUGRIFF AUF PATIENTENDATEN
PROCÉDÉ ET SYSTÈME D'ACQUISITION DE DONNÉES RELATIVES À UN PATIENT

(30) Priority: 31.05.2011 DK PA201100412
(43) Date of publication of application: 30.11.2022
(62) Document number(s) of the earlier application(s) in accordance with Art. 76 EPC: 12793858.7 / 2 713 856
(73) Proprietor: Radiometer Medical ApS, 2700 Brønshøj (DK)
(72) Inventor: CARLBERG, Anders, 211 21 Malmø (SE); OLOFSSON, Niclas, 217 75 Malmø (SE)
(74) Representative: Maiwald GmbH

(56) References cited:
- WO-A1-2011/050668
- US-A- 5 626 144
- US-A1- 2012 072 536

## Description

### TECHNICAL FIELD

Embodiments of the methods, product means, systems and devices disclosed herein relate to the field of acquisition and processing of patient-related data such as test and measurement results and/or other clinical data.

### BACKGROUND

Within the field of clinical analysis, a wide variety of electronic devices are known for the acquisition and registration of patient-related data. In particular, devices are known that allow clinical personnel to acquire test and/or measurement results related to a given patient at the point of care. Such electronic devices include devices for performing various forms of clinical tests and/or analysis, such as the measurement of physiological parameters of a patient.

In modern clinical environments, such electronic devices are widely used and there is a trend of moving more and more tests from a central laboratory to the actual point of care (POC). Even though this has a number of advantages, it also involves a number of challenges.

For example, the operational environment in which POC electronic devices are operated is less controllable than the environment of a central laboratory, e.g. in terms of controlling the personnel operating the devices. Furthermore, any electronic device may be operated by a number of different operators during the course of a day.

The data acquired by an electronic device often needs to be transferred to and recorded by a central data processing system, e.g. a server system implementing a patient database. It is desirable that this transfer is performable securely, reliably and efficiently.

As electronic devices are frequently used for acquiring data relating to different patients, it is desirable to reduce the risk of patient mix-up and to ensure that correct data is registered in a patient database.

US 5626144 discloses a system for monitoring and reporting medical information of patients suffering from chronic diseases such as diabetes or asthma. This prior art system includes a stand-alone monitor for storing data records comprising measured values and time stamps and for transmitting the records to a remote reporting unit. The remote reporting unit includes a relational database that comprises patient records each including a unique ID code pairing a patient and a remote sensor. The database is updated when records are downloaded and it allows the generation of chronological reports of status information of a physiological characteristic. Hence, even though this prior art system allows the remote collection of data from a monitoring device used by an individual patient in the course of long-term management of a chronic disease, it remains a problem to reliably record patient-related data in a hospital or other healthcare facility where multiple electronic devices are frequently used by different operators and to obtain data related to different patients.

US 2003/0065653 discloses a system for storing and retrieving medical records.

According to this prior art system, when a medical device is first used on the system, its own identification (manufacturer, and device ID number) is automatically registered with the system. A patient receives an ID number, the first time the patient receives an ECG or is x-rayed by equipment that is registered with this prior art system. Thus the first time a patient is examined via medical equipment of this prior art system, a universal record of not only the equipment, but also the patient is automatically created. The patient is provided with a patient identification card, and from that point on, the patient identification card is "registered" within the system. Even though this system allows for a central registration of patients and medical devices, it requires the individual medical devices to be adapted for use with the system.

WO2011/050668 A1 discloses a method, device and system for remotely obtaining physiological detection data of a user. The method includes: receiving relevant detection information transmitted by a portable device, and the relevant detection information contains identity information of the user and device positioning information; sending an instruction of starting detecting to a corresponding medical detection device according to the device positioning information; receiving the physiological detection data of the user sent by the medical detection device, and the physiological detection data of the user is relevant to a device identity (ID) of the medical detection device; binding and sending the physiological detection data of the user and the relevant identity information of the user according to the relevant information between the identity information of the user and the device identity (ID) of the medical detection device.

However, in many clinical environments, there is a large variety of types of electronic devices with varying capabilities, e.g. in terms of data management, processing power, etc. At the same time, it is desirable to keep the requirements imposed on the capabilities of electronic devices limited so as to be able to manufacture them at a reasonable cost.

Generally it is desirable to ensure the quality of the acquired data, and to be able to verify compliance with operating procedures at the clinic at which the electronic devices are operated.

### SUMMARY OF THE INVENTION

The invention provides a method of registering patient-related data as defined by appended claim 1, a system as defined by claim 18, and a computer program product as defined by claim 19. Embodiments of the invention are set out in the dependent claims.

### Summary of related disclosure

Hence, embodiments of the method described herein provide a reliable association of the acquired data to a particular patient without having to rely on patient data management or similar capabilities of the electronic device. As a matter of fact, the electronic device is not required to receive or store information identifying the patient. In particular, embodiments of the method described herein provide a reliable association of patient-related data with a patient identifier, where the data has been acquired by an electronic device. It has been realised that this association may be reliably performed merely based on a device identification data item identifying the electronic device, a patient identification data item identifying the patient, a time stamp identifying the time at which the data acquisition occurred, and one or more time stamps associated with one or more of the identification data items, e.g. one or more time stamps indicative of the times the device and/or the patient have been registered. Furthermore, embodiments of the method described herein allow for an establishment of a unique link between the acquired data and a patient even at a later point in time, e.g. if the acquired data is not transferred from the electronic device to the data processing system right away but rather at a later point in time. For example, some electronic devices that do not have any wireless communications interfaces may store the acquired data until the electronic device is connected to the data processing system via a wired connection, a docketing station, or some other suitable interface.

In embodiments of the method described herein, an establishment of an association between the received patient-related data and an identified patient is performed on the basis of at least the received device identification data item, the received patient identification data item, the received one or more identification time stamps and the received acquisition time stamp. It is an advantage of embodiments of the method described herein that they may be adapted to different standard operating procedures according to which health care personal may perform the data acquisition and patient registration. To this end, some embodiments of the association step may compare the acquisition time with a first time identified by a first one of the one or more identification time stamps, and the association may then be based on a result of this comparison. For example, comparing may comprise determining whether the acquisition time lies within a predetermined data acquisition time interval determined from at least said first identification time stamp; and associating may comprise associating, if the acquisition time lies within said predetermined data acquisition time interval, the patient-related data with the patient identification data item. The data acquisition time interval may be a time interval starting at said first time and having a predetermined length. Hence, the first time may be a start trigger for a time interval during which acquired data will be associated to the registered patient. Alternatively, the data acquisition time interval may extend a first predetermined period prior to the first time and a second predetermined period after the first time. Hence, the acquisition time interval may be defined based on a single time stamp.

In some embodiments, receiving patient-related data to be registered and an acquisition time stamp further comprises obtaining a device identifier of the electronic device from which the patient-related data is received; and associating the received patient-related data with an identified patient is further based on said obtained device identifier of the electronic device from which the patient-related data is received. In particular, received patient-related data may be associated with a patient identifier that has been received in a set of identification data items including a device identification data item which matches the obtained device identifier of the electronic device from which the patient-related data has been received. Hence, the association based on time stamps described herein may even be reliably performed when different electronic devices are concurrently used for acquiring patient-related data.

Accordingly, associating the received patient-related data with an identified patient may comprise
- determining one or more received sets of identification data items each comprising a device identification data item that matches the obtained device identifier of the electronic device from which the patient-related data has been received; and
- associating the received patient-related data with the patient identification data item of one of the determined sets of identification data items based on at least the received one or more identification time stamps and the received acquisition time stamp.

In some embodiments, receiving a set of identification data items and one or more identification time stamps comprises receiving at least said first and a second identification time stamp, the second identification time stamp identifying a second time; and the data acquisition time interval is a time interval starting at said first time and ending at said second time. Hence, the data acquisition time interval is determined by two time stamps associated with respective identification data items. In particular, the first and/or the second time may be an identification time at which one of the identification data items has been obtained. Hence, in this embodiment the start and/or the end of the data acquisition time interval is defined by a registration event, e.g. by the operator scanning or otherwise registering the patient, the electronic device, and/or the like. Consequently, the acquisition time interval is defined by the actual registration process and automatically adapted to the chosen registration procedure and speed.

In some embodiments, determining the data acquisition time interval may comprise determining the data acquisition time interval as a time interval from an earliest one to a latest one of the identification times indicated by the received set of identification time stamps. Hence, the association of the acquired data is based on a time interval established by the time interval between an initial registration of one of the identification data items, e.g. the device identification data item, and the registration of the last one of the set of identification data items, e.g. the patient identification data item. Hence, in some embodiments the device identification data item, the patient identification data item, and optionally additional identification data items such as an operator identification data item, may be registered in any order. The earliest and the latest of the registrations define a time interval that is subsequently used by the data processing system to associate the acquired data with a given patient. Accordingly, as long as the operator performs the registration of at least one of the identification data items prior to the data acquisition, and the registration of at least another one of the identification data items after the data acquisition, the data processing system may uniquely associate the acquired data with a given time interval, and thus with the corresponding patient identification data item.

In some embodiments, the first and/or the second time is a time at which a predetermined trigger event has occurred. Hence, in some embodiments, the start and/or end of the data acquisition time interval may be determined by a predetermined trigger event, e.g. a predetermined operator input to the registration device, pressing a button, obtaining one of the identification data items, etc. or a combination thereof. Other examples of such an event include the receipt of a message by the registration device from an external system, e.g. a host system. Yet another example of such an event may be the expiry of a timer of the registration device.

The device identification data item and the patient identification data item are comprised in a set of identification data items, thus allowing them to be associated with one another as belonging to the same set, i.e. the same data acquisition event. In some embodiments, the data processing system may receive the set of identification data items as identification data items that are explicitly associated with another, e.g. by receiving a common data record including the set of identification data items. Alternatively, in some embodiments, receiving the set of identification data items comprises receiving a plurality of identification data items (which may not yet be explicitly associated with one another as belonging to respective data acquisition events) and determining said set of identification data items from said received plurality of received identification data items as a set of identification data items associated with said first identification time stamp. For example, the data processing system may identify a (sub-)set of identification data items to be associated with a common data acquisition event based on the relative timing of the individual registrations of identifiers and/or based on the sequential order of the individual registrations, and/or whether or not a complete set of a number of required identifiers has been registered, e.g. a patient identifier, a device identifier, and an operator identifier. Hence, embodiments of the method described herein may be adapted to different capabilities of registration devices and easily matched to different standard operating procedures.

As mentioned above, the objects identified by the identification data items of the identification data items include the electronic device and the patient to which the patient-related data relates. In some embodiments the set of identification data items further comprises an operator identification data item identifying an operator of the electronic device, thus increasing the safety, quality, and compliance of the registration process by allowing the system to verify whether the operator is authorized and/or trained to operate the electronic device.

The identification data items may be embodied as any suitable data structure indicative of an object - e.g. a patient, and electronic device, an operator, or the like - to be identified. For example, the identification data item may comprise a suitable identifier for identifying an object. Preferably, the identifier uniquely identifies an object. In addition to an identifier, the identification data item may comprise additional information such as a time stamp indicative of a registration time at which the identifier has been obtained, i.e. the time at which the respective object has been registered.

In some embodiments, receiving an acquisition time stamp from the electronic device further comprises receiving, from the electronic device, a control time stamp indicative of a current time determined by the electronic device; and correcting the acquisition time stamp based on a comparison of the control time stamp with a current time determined by the data processing device. Hence, even if the acquisition time may be determined by the electronic device based on an internal clock that may not be synchronised with the time registered by the data processing system, the data processing system may correct the received time stamp so as to be consistent with the time registered by the data processing system.

The present disclosure relates to different aspects including the method described above and in the following, corresponding apparatus, systems, and products, each yielding one or more of the benefits and advantages described in connection with the above-mentioned method and/or one of the other aspects, and each having one or more embodiments corresponding to the embodiments described in connection with the above-mentioned methods and/or one of the other aspects.

In particular, disclosed herein are embodiments of a data processing system adapted to perform the method described above and in the following. The data processing system may be a suitably programmed computer such as a server computer, a desktop computer, and/or the like, and/or a data processing system comprising several computers, e.g. computers connected with each other via a suitable computer network.

Furthermore, disclosed herein are embodiments of a system for acquiring patient-related data, the system comprising a data processing system, an electronic device and a registration device as described herein. The terms electronic device and registration device are intended to comprise any device comprising processing means for data processing. In particular, the above terms are intended to comprise any electronic equipment, portable radio communications equipment, and other handheld or portable devices, personal computers or other computers or data processing systems. Embodiments of the electronic device and/or the registration device may further comprise a respective communications interface adapted for communicating data to the data processing system, e.g. via a wired or a wireless communications channel, such as a local area network, a wireless local area network, a wide area network, an internet, a telecommunications network such as a cellular communications network, a short range wireless communications interface, a serial or parallel interface, a USB interface, a Bluetooth interface, and/or the like.

Embodiments of the electronic device may further comprise data acquisition means for acquiring patient-related data such as test data, measurements of physiological parameters, detected types and/or dosages of a medication, etc. Embodiments of the registration device may further comprise input means for receiving identifiers; for example, the input means may include a reader for reading a machine readable code such as a barcode scanner, a reader for reading radio-frequency identification (RFID) tags or labels, a user-interface allowing manual input of identifiers, a camera, or any other suitable input means. Generally, embodiments of the electronic device may include a medical device, such as a clinical instrument for performing clinical tests and/or analysis, a drug dispensing device, and/or another medical device for clinical use.

The term portable radio communications equipment includes all equipment such as mobile terminals, e.g. mobile telephones, pagers, communicators, electronic organisers, smart phones, personal digital assistants (PDAs), handheld computers, or the like.

Embodiments of the electronic device and/or the registration device may further comprise respective storage media, e.g. a hard disc, an optical disc, a compact disc, a DVD, a memory stick, a memory card, an EPROM, and/or the like. Embodiments of the electronic device may further comprise means for establishing a current time, e.g. an internal clock and/or by receiving time information from an external source such as the data processing system and/or a communications network.

It is noted that the features of the methods described herein may be implemented in software and carried out on a data processing system or other processing means caused by the execution of program code means such as computer-executable instructions. Here and in the following, the term processing means comprises any circuit and/or device suitably adapted to perform the above functions. In particular, the above term comprises general- or special-purpose programmable microprocessors, Digital Signal Processors (DSP), Application Specific Integrated Circuits (ASIC), Programmable Logic Arrays (PLA), Field Programmable Gate Arrays (FPGA), special purpose electronic circuits, etc., or a combination thereof.

Hence, according to another aspect, a computer program comprises program code means adapted to cause a data processing system to perform the steps of the method described herein, when said computer program is run on the data processing system. For example, the program code means may be loaded in a memory, such as a RAM (Random Access Memory), from a storage medium or from another computer via a computer network. Alternatively, the described features may be implemented by hardwired circuitry instead of software or in combination with software.

### BRIEF DESCRIPTION OF THE DRAWINGS

The above and other aspects will be apparent and elucidated from the embodiments described with reference to the drawing in which:
Fig. 1 shows a schematic block diagram of an example of a system for acquiring patient-related data.
Fig. 2 shows a schematic functional block diagram of an embodiment of a system for acquiring patient-related data.
Fig. 3 shows a flow diagram of an example of a method of acquiring patient-related data.
Fig. 4 illustrates an example of the operation of an embodiment of a system for acquiring patient-related data.
Figs. 5 and 6 illustrate different embodiments of a method of acquiring patient-related data.

### DETAILED DESCRIPTION

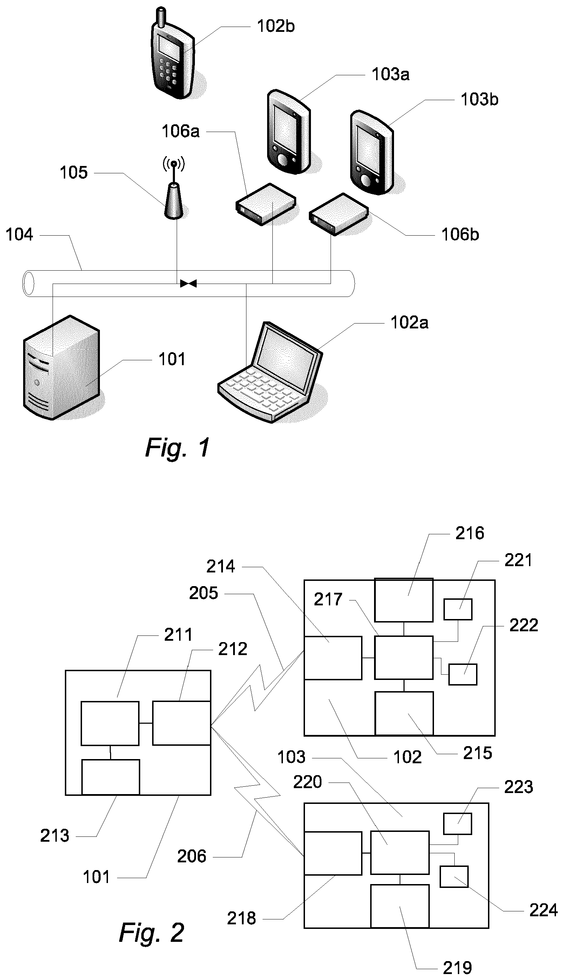

Fig. 1 shows a schematic block diagram of an example of a system for acquiring patient-related data. The system comprises a server computer 101 or other suitable data processing system suitably programmed to store and maintain medical records of patients, e.g. in a suitable database system. The server computer 101 is connected to a computer network 104, e.g. a local area network (LAN). The computer network 104 may comprise a wireless access point 105 or other suitable wireless interface device allowing electronic devices to wirelessly connect to the computer network 104. The system further comprises a number of registration devices 102a, 102b, connected or connectable to the computer network 104. In the example of fig. 1, the system comprises two registration devices, one registration device 102a being connected to the computer network via a wired connection, e.g. via a local area network interface circuit, and one registration device 102b being wirelessly connected, e.g. via wireless access point 105, to the computer network 104. It will be appreciated, however, that embodiments of the system described herein may comprise any number of registration devices, each being connectable to the computer network via a suitable communications interface. The system further comprises a number of electronic devices 103a and 103b. In the example of fig. 1, the electronic devices are connectable to the computer network via respective docking stations 106a and 106b. However, it will be appreciated that embodiments of the system described herein may comprise any number of electronic devices. The electronic devices may be identical with each other or different from each other, and they may be connectable to the computer network in a variety of different ways, e.g. by means of a suitable wired and/or wireless connection.

Each of the registration devices may be a suitable portable or stationary computer or other computing device, such as a laptop computer, a desktop computer, a handheld computer such as a PDA, a smartphone, a tablet computer, etc.

Fig. 2 shows a schematic functional block diagram of an embodiment of a system for acquiring patient-related data. The system comprises a data processing system 101 (in the following also referred to as host system) suitably programmed to store and maintain medical records of patients, a registration device 102, and an electronic device 103. As mentioned above, even though fig. 2 only illustrates a single registration device and a single electronic device, embodiments of the system may include respective pluralities of these devices. It will also be appreciated that the functionality of the host system 101 may be distributed among a plurality of physical devices, e.g. a plurality of server computers and/or a plurality of database systems.

The registration device 102 is connectable to the host system 101 via a suitable communications link 205 allowing data communication between the host system 101 and the registration device 102. To this end, the registration device and the host system comprise respective communications interfaces 214 and 212, allowing data communication via the communication link 205. Similarly, the electronic device 103 is connectable to the host system 101 via a suitable communications link 206 allowing data communication between the host system 101 and the electronic device 103. To this end, the electronic device and the host system comprise respective communications interfaces 218 and 212, allowing data communication via the communication link 206. The communications links 205 and 206 may be implemented using the same or different communications technologies, e.g. the same or different computer networks. Accordingly, the communications interface 212 of the host system may be embodied as a single interface device or as a plurality of interface devices supporting respective communication technologies, e.g. a network adapter for a wired connection to a local area network and a radio-frequency transmitter for connecting the host computer with other devices via a short-range radio-network. Generally, examples of suitable communication interfaces include a wired or wireless network adapter, a radio-frequency communications interface allowing communication via a telecommunications network such as a cellular communications network, a radio-frequency communications interface allowing communication via a short-range wireless communications interface, a serial or parallel interface adapter, a USB port, and/or the like.

The host system 101 further comprises a processing unit 211 such as a suitably programmed CPU of a computer or other suitable processing means, communicatively coupled to the communications interface 212. The host system 101 further comprises a data storage device 213 communicatively coupled to the processing unit 211, e.g. a database system or other data storage device for storing patient related data.

The registration device 102 comprises a processing unit 217 such as a suitably programmed CPU of a computer or other suitable processing means, communicatively coupled to the communications interface 214. The registration device further comprises a data input unit or circuitry 215 for receiving identification data. The data input unit 215 is communicatively connected with the processing unit 217. Examples of the data input unit or circuitry include a reader for reading a machine-readable code such as a barcode scanner, a reader for reading radio-frequency identification (RFID) tags or labels, a user-interface allowing manual input of identifiers, a camera, or any other suitable input means. The registration device may further comprise a user-interface 216, e.g. a display allowing the registration device to present output to a user of the registration device, e.g. by means of a graphical user interface. The registration device may further comprise a timer circuit 221, e.g. a clock or other suitable functionality for determining the current time, and a suitable data storage device 222 for storing registration data received via the data input unit, e.g. a RAM, an EPROM, a hard disk, etc.

The electronic device 103 comprises a processing unit 220, such as suitably programmed microprocessor or other suitable processing means, communicatively coupled to the communications interface 218. The electronic device 103 further comprises a data acquisition device 219 communicatively coupled to the processing unit 220 for acquiring patient-related data such as test data, measurements of physiological parameters, detected types and/or dosages of a medication, etc. The registration device may further comprise a timer circuit 223, e.g. a clock or other suitable functionality for determining the current time, and a suitable data storage device 224 for storing data acquired by the data acquisition device 219, e.g. a RAM, an EPROM, a hard disk, etc. Examples of an electronic device include a meter or analyzer for measuring patient-related data, e.g. a blood glucose meter, a blood gas analyser, an analyzer for measuring cardiac, coagulation, infection and/or pregnancy markers, and/or the like.

Embodiments of operating a system for acquiring patient-related data, e.g. a system as described in fig1. and/or 2, will now be described in more detail.

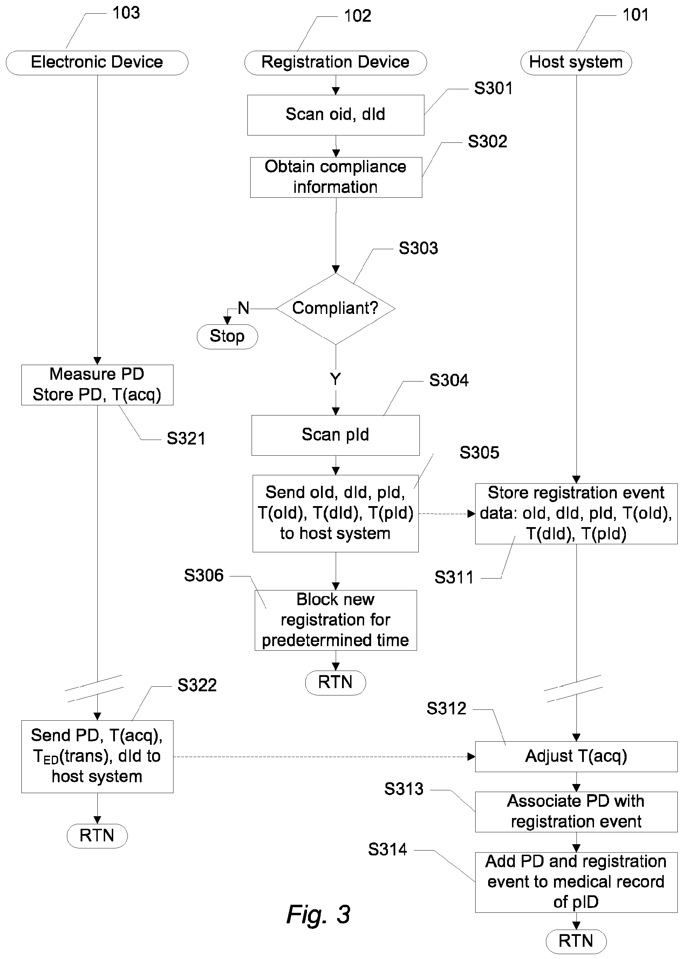

Fig. 3 shows a flow diagram of an example of a method of acquiring patient-related data. The process is performed by a system comprising a host system 101, a registration device 102, and an electronic device 103, e.g. as described in connection with figs 1 and/or 2.

The process may be regarded as comprising two sub-processes, a first one performed by the registration device and the host system, and a second sub-process performed by the electronic device and the host system. The first sub-process comprises steps S301-S306 and step S311, while the second sub-process comprises steps S321-S322 and steps S312-S314.

The first sub-process is initiated in step S301 by the registration device 102 obtaining an operator identifier *old* and a device identifier *dld,* the operator identifier identifying the operator of the electronic device 103, and the device identifier identifying the electronic device 103. The operator may be a nurse or other health care professional authorised to operate the electronic device 103. For example, the registration device may obtain the identifiers by scanning respective barcodes associated with the operator and the electronic device, respectively. For example, the operator may carry an identification card having printed on or otherwise applied to it a barcode, and the electronic device may have applied to it a label carrying a barcode. It will be appreciated that the electronic device may obtain the identifiers in any other suitable way, e.g. by means of RFID tags attached to the electronic device and the operator's cloths or an ID card. The registration device may be a portable device carried by the operator. Alternatively, the registration device may be a stationary device, e.g. located in a room of a hospital used for examining patients or where samples are obtained from patients. The registration device further registers the time *T(old*) at which the operator identifier has been obtained, and the time *T(dld)* at which the device identifier has been obtained. The registration device associates the registered times with the respective obtained identifiers and may store the identifiers with their associated times in a local memory of the registration device, e.g. as respective identification data records, e.g. record comprising an identifier and an associated time stamp. The registration device may acknowledge a successful scan, e.g. by generating an audible sound and/or by displaying a suitable message on a display of the registration device, e.g. *"Welcome Nurse B"* in response to scanning the operator ID of nurse B.

In step S302, based on the obtained identifiers, the registration device obtains compliance information indicative of a compliance status of the electronic device 103 and/or a compliance status of the operator, e.g. information as to whether operation of the electronic device by the operator is in compliance with administrative rules of the hospital or health care centre at which the process is performed. For example, the electronic device may obtain information about the time remaining until the next required calibration or maintenance of the electronic device, and/or authorisation information indicating whether the operator is authorised (e.g. qualified and properly trained) to operate the electronic device. The registration device may obtain the compliance information from a local compliance database stored in the registration database. For example, the local compliance database may regularly, e.g. daily, be updated by synchronising a central compliance database which may reside on the host system 101 or another suitable data processing system. Alternatively the registration device may obtain the compliance information from an external system, e.g. the host system 101. To this end, the registration device may communicate a compliance query including the operator identifier and/or the device identifier to the external system, e.g. to the host system 101 via communications link 205. The external system may return a compliance response indicative of whether operation of the electronic device by the operator is in compliance with the administrative rules of a hospital.

In step S303, the registration device determines, based on the obtained compliance information, whether operation of the electronic device by the operator is compliant with current administrative rules. If this is not the case, the registration device informs the operator (e.g. by an audible signal, a display of a suitable error message, and/or the like) about the lack of compliance, and terminates the process. Otherwise, the process proceeds at step S304, optionally after displaying the obtained compliance information on a display of the registration device, optionally together with additional information, e.g. when the next calibration of the device is due, when the operator is scheduled to receive further training in respect of the device, etc. For example, the device may display: "*OK, You are certified for device X until May 17, 2011, QC: OK* - *next QC before May 12 07:00. CAL: OK* - *next CAL before May 11 17:00".* It will be appreciated that, in some embodiments, the process may not include the compliance test of steps S302 and S303, i.e. the process may directly proceed from step S301 to step S304. In yet other embodiments, a determination of lack of compliance may merely result in a warning message without termination of the process. It will be appreciated that, in embodiments that do not include a compliance test of the authorisation of the operator, the process may not require obtaining of the operator identifier.

In step S304, the registration device obtains a patient identifier *pld* identifying a patient from which the operator intends to acquire (or already has acquired) patient-related data by means of the electronic device 103. For example, the registration device may obtain the patient identifier in a similar fashion as the operator and device identifiers. To this end, the patient may carry or wear a wrist band, an ID card, or the like, including the identifier, e.g. in the form of a barcode and RFID tag, or the like. It will be appreciated that the patient identifier may also be an identifier identifying another object (e.g. a hospital bed) that is uniquely associated with the patient. The registration device further registers the time *T(pld)* at which the patient identifier has been obtained, and associates the registered time with the obtained patient identifier. The registration device may store the patient identifier with its associated time in a local memory of the registration device, e.g. as a patient identification data record comprising the patient identifier and an associated time stamp. The registration device may acknowledge a successful scan, e.g. by generating an audible sound and/or by displaying a suitable message on a display of the registration device, e.g.: "*Patient ID: 1236556. Name: Peter Hansen Sex: Male."*

In step S305 the registration device sends the patient identifier, the device identifier, and the operator identifier together with the respective associated time stamps indicative of the times at which the respective identifiers have been obtained to the host system 101, e.g. in the form of respective identification data records described above. For example the registration device may forward the identifiers and time stamps to the host system via communications link 205 of fig. 2. Alternatively to forwarding all identifiers and time stamp in a single transmission, the registration device may forward the operator identifier and the device identifier, including their associated time stamps, to the host system immediately after the registration device has obtained the respective identifier, thereby avoiding the need for internally storing the identifiers and time stamps. Hence, in such an embodiment, the registration device at this point only needs to send the patient identifier and the associated time stamp to the host system. In an embodiment, where the registration device forwards each identifier to the host system immediately after the registration device has obtained the identifier, the registration device may not need to generate and forward a time stamp associated with the identifier. In such an embodiment, the host system may generate and store a suitable time stamp upon receipt of each identifier.

In step S311, the host system receives and stores the registration identifiers and associated time stamps. In one embodiment, the host system may store the identifiers as separate identification data records, each comprising an identifier and an associated time stamps. It will be appreciated that the identification data records may comprise additional information, e.g. an identifier identifying the registration device from which the identifier has been received. For example, the registration device may transmit such an identifier identifying the registration device together with the identifiers. In alternative embodiments, the host system may store the identifiers in a data record identifying a registration event where each registration event comprises a predetermined set of identifiers. In the present example, this set of identifiers may comprise the operator identifier, the device identifier and the patient identifier. Each registration event may further comprise one or more time stamps, e.g. a time stamp associated with one of the identifiers, or respective time stamps associated with some or each of the identifiers. In both embodiments, the set of identifiers comprising the patient identifier the operator identifier, and the device identifier are related to a common registration event.

After sending the identifiers and their associated time stamp, the process continues at step S306 where the registration device prevents the scanning/obtaining of additional identifiers for a predetermined time-out period, thereby providing a minimum time interval separating the time stamps associated with identifiers that relate to different registration events. For example, the registration device may prevent registration of further identifiers for a period sufficiently long to allow the host system to reliably associate measurement events and registration events, as will be described in greater detail below. Generally, the duration of the time-out period may depend on the lowest accuracy and resolution of the time stamps generated by any of the registration devices and the electronic devices of the system. For example, when the resolution of time stamps is minutes, the registration device may prevent registration of further identifiers for a period chosen between 1 min. and 3 min. During that period, the registration device may display a message requesting the operator to await the end of the time-out. The registration device may even display a countdown indicating the remaining time-out period. In some embodiments, the registration device may allow the operator to override the time-out and/or to cancel the previous registration event and to immediately start a new registration event. In the latter case, the registration device may send a request to the host system requesting deletion of the latest set of identifiers received by the host system from the registration device. In some embodiments, the prevention of obtaining additional identifiers for a pre-determined time-out period may be omitted. Instead, when the process obtains the device identifier *dld* in step S301, the process may determine the time resolution with which the identified device determines time stamps (e.g. by querying a database of devices using the device identifier as a key). If the time elapsed since the most recent previous scanning/obtaining of the same device identifier is smaller than the time resolution of the electronic device, the process may stop with a suitable error message or perform another suitable step, e.g. continue but display a warning message.

The second sub-process is initiated by the electronic device 103 obtaining patient-related data *PD* in step S321 and generating a corresponding time stamp *T(acq)* associated with the patient-related data and indicative of the time at which the patient-related data has been obtained. For example, if the electronic device is a measuring device, e.g. for measuring one or more physiological parameter of the patient, the patient-related data may comprise one or more measured values indicative of the one or more physiological parameters. Similarly, if the electronic device is a dispensing apparatus, e.g. for dispensing predetermined amounts of medicine, the patient-related data may include the type and/or amount of dispensed medicine. The electronic device may acknowledge successful data acquisition, e.g. by generating an audible sound and/or by displaying a suitable message on a display of the electronic device, e.g.: "*<time stamp* as *recorded> Your result will be transmitted to the patient record next time the instrument is placed in the docking station."*

In subsequent step S322, the electronic device 103 forwards the acquired data *PD*, the associated time stamp *T(acq)* to the host system 101. The electronic device may further transmit its own device identifier *dld* together with the patient-related data *PD* and the time stamp *T(acq),* thus allowing the host system to identify the electronic device from which the patient-related data originates. Alternatively, the host system may obtain the device identifier of the electronic device that has forwarded the patient-related data in a different way, e.g. by assigning different communication ports of the host system to different electronic devices, and by determining (by the host system) over which port the patient related data has been received. The electronic device may forward the acquired patient-related data immediately after it has been acquired or at a later point in time, e.g. several minutes or even hours later. An immediate transmission may be possible, if the electronic device comprises real-time communications capabilities, e.g. via a wireless communications link between the electronic device and the host system. A delayed transmission may occur when the electronic device only communicates with the host system periodically, e.g. at regular or even irregular time intervals between opportunities to transmit data. For example, many electronic devices do not comprise wireless communications capabilities. Examples of such devices include hand-held meters that may be placed in a cradle or docking station which is connected to a computer network. Such devices may only be able to transmit data when placed in its cradle or docking station, e.g. after the operator has acquired a large amount of data, typically from different patients and over an extended period of e.g. several hours or even an entire day. Other examples may include meters that store data on a storage medium, e.g. a memory card, such that communication of the acquired data involves the operator removing the storage medium from the meter and inserting it in a card reader that is connected to the host system.

In any event, when the acquired patient-related data is forwarded to the host system, the host system may store the received patient-related data and associated time stamp. In some embodiments, the host system may adjust (step S312) the received time stamp *T(acq)* for any detected inaccuracy of synchronisation between the time as measured by the electronic device, and the time measured at the host system. To this end, the electronic device may further transmit a reference time stamp *T_{ED}(trans)* together with the patient-related data *PD* and the time stamp *T(acq),* where the reference time stamp may be indicative of the current time (as determined by the electronic device) at which the electronic device transmits the patient-related data. Upon receipt of the data, the host system may compare the received reference time stamp with the local time of receipt *T_{HS}(rec)* determined by the host system. The host system may calculate the difference *DT = T_{HS}(rec) -T_{ED}(trans)* and, assuming that the difference *DT* is due to a lack of synchronization of the internal clocks of the electronic device and the host system, adjust the time stamp *T(acq)* accordingly, e.g. according to *T(acq) -> T(acq)+DT.* Alternatively, the host system, and/or the registration device may include functionality allowing the host system or the registration device to set the clock of the electronic device, e.g. by sending a suitable request to the electronic device, if the electronic device supports such requests. Yet alternatively, for those devices where it is not possible to adjust the clock, the host system or registration device may warn the operator about the mismatch allowing the operator to manually adjust the time on the electronic device.

It will be appreciated that the first- and second sub-processes are asynchronous in the sense that there is no direct link between the initiation of both processes. Each process is normally initiated by the operator when the operator initiates registration of the first identifier (step S301) and when the operator initiates the obtaining of the patient-related data (step S321), respectively. Hence, the system cannot control the relative timing of the data acquisition step S321 and steps S301 and S304 at which the identifiers are obtained. It will be appreciated that the order of these steps will often be prescribed by standard operation procedures of the hospital or health care center which may prescribe a predetermined order of steps to be performed by the operator. Hence, the host system may receive a plurality of patient-related data items obtained at different times by the same electronic device but related to different patients.

Accordingly, in subsequent step S313, the host system associates the patient related data to a patient identifier, based on the device identifier *dld* (e.g. based on a comparison of the device identifier obtained from the electronic device that has forwarded the patient-related data with the device identifier that was registered in connection with the registration of the patient identifier), on the (optionally adjusted) time stamp *T(acq)* of the patient-related data, and on one or more time stamps of a previous registration event performed by the registration device, as described herein. Embodiments of how the host system may use the time stamps to make this association will be described in greater detail below.

Subsequently, in step S314, the host system may add the received patient-related data, optionally together with the associated identifiers of the operator and/or the electronic device, to a patient record associated with the patient identifier to which the host system has associated the patient-related data. Additionally or alternatively, the host system may forward the results to a different system for storage and/or further processing.

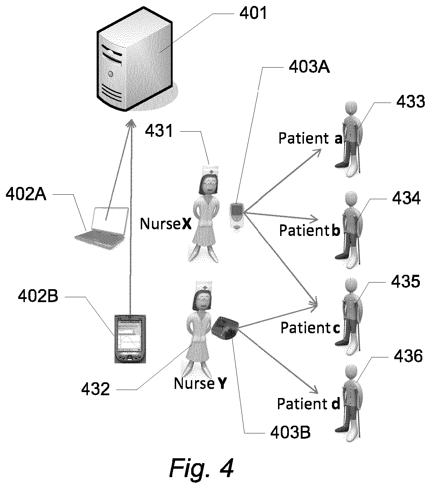

Fig. 4 illustrates an example of the operation of an embodiment of a system for acquiring patient-related data. In this example, the system is operated by nurses X and Y (designated by reference numerals 431 and 432, respectively) of a hospital to obtain measurements to be associated with patients *a*-*d*, designated by reference numerals 433-436, respectively. In this example, nurse X carries electronic device 403A, while nurse Y carries electronic device 403B. The system further comprises host system 401 and registration devices 402A and 402B connected to the host system 401.

In the following, an exemplary process of obtaining the measurements will be described as a sequence of steps 1) through 17):
1) Nurse X takes Device 403A from e.g. its docking station (not explicitly shown in fig. 4)
2) Nurse X scans a device identification barcode of device 403A, and her own barcode using the registration device 402A.
   a. The registration device 402A connected to the host system 401 determines, based on the scanned barcodes, whether device 403A is in compliance, and issues a warning if device 403A is not in compliance (e.g. due to missing quality control activities) or if nurse X is not trained or retraining is overdue.
   b. If the device 403A and nurse X are in compliance, device 403A may be used for testing, and the process continues.
3) Nurse X performs a measurement on patient a using device 403A.
4) Nurse X scans patient a's barcode using the registration device 402A.
5) Nurse Y takes Device 403B from e.g. its docking station.
6) Nurse X performs steps 2, 3 and 4 for Patient b, using registration device 402A and electronic device 403A.
7) Nurse X performs steps 2,3 and 4 for Patient c using registration device 402A and electronic device 403A.
8) Nurse Y performs step 2 on Device 403B and using registration device 402B.
9) Nurse Y performs a measurement on Patient c, using Device 403 B.
10) Nurse Y scans Patient c using the registration device 402B.
11) Nurse Y performs steps 8, 9 and 10 on Patient d.
12) Nurse X returns device 403A to its docking station.
13) Nurse Y returns device 403B to its docking station.
14) Device 403A establishes a connection with the host system (via its docking station) and sends three messages with measuring results to the host system. Each of the messages includes the following information:
   a. Sample time *T(acq)* (i.e. the time at which the measurement result has been obtained)
   b. Device ID *dld* identifying the electronic device used to obtain the measurement result
   c. Measurement result *PD*
15) Device 403B establishes a connection with the host system (via its docking station) and sends two messages with measuring results to the host system. Each of the messages includes the above specified information, i.e. sample time, device ID, and measurement result.
16) The host system 401 maps the sample times and device IDs received in the messages from Devices 403A and 403B, respectively, with the time-of-scanning time stamps, and device ID's obtained by the scanning performed in steps: 2, 4, 6, 7, 8, 10 and 11, thereby associating the measurement data to the patient IDs.
17) The host system stores the five measurement results with the corresponding patient identifiers of patients *a*-*d*, respectively, and/or forwards the results to a separate system for storage and/or further processing.

It will be appreciated that the sample time stamp *T(acq)* and the Device ID *dld* together generate a unique identifier allowing the host system to associate the measurement data to the scanned barcodes.

By performing two of the ID registration steps prior to the actual measurement and by performing the patient registration after the measurement, the host system may create a time interval within which the host system can map the measurement belonging to the registration.

For example, in the above example of fig. 4, steps 2), 3) 4) and 6) result in the following sequence of registrations:
1) Scan operator ID (Nurse X)
2) Scan device ID (Device 403A)
3) Perform measurement (Patient a)
4) Scan Patient ID (Patient a)
5) Scan operator ID (Nurse X)
6) Scan device ID (Device 403A)
7) Perform measurement (Patient *b*)
8) Scan Patient ID (Patient *b*)

Hence, upon receipt of the time stamps identifying the timing of the above registrations, the host system may establish a first time interval from the earliest to the latest ID scan associated with the measurement on patient a, i.e. from registration 1) to registration 4). Similarly, the host system may establish a second time interval from the earliest to the latest ID scan associated with the measurement on patient *b*, i.e. from registration 5) to registration 8). From the time stamps of measurements 3) and 7), respectively, the host system may thus determine that the measurement on patient a (registration 3)) was performed in the time interval between registrations 1) and 4), and thus associate the measurement on patient a with the patient ID registered in registration 4). Similarly, the host system may associate the measurement on patient b (registration 7)) with the patient ID obtained in registration 8).

It will be appreciated that, in the above embodiment, the above association does not require a particular sequential order in which the operator ID, the device ID, and the patient ID are scanned, as long as the measurement is performed after the scanning of the first one of the IDs and before the scanning of the last one of the IDs. Furthermore, it will be appreciated that the scanning of the operator ID is not even required for the host system to be able to establish a time interval and to associate the measurement with a patient ID. However, by scanning the operator ID and the device ID before the measurement, a compliance check may be performed as described above.

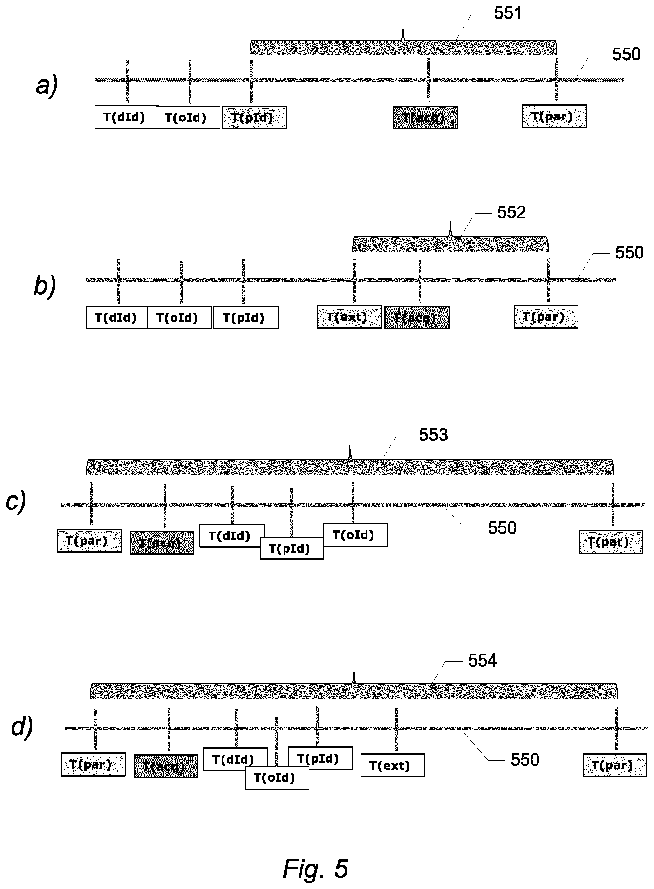

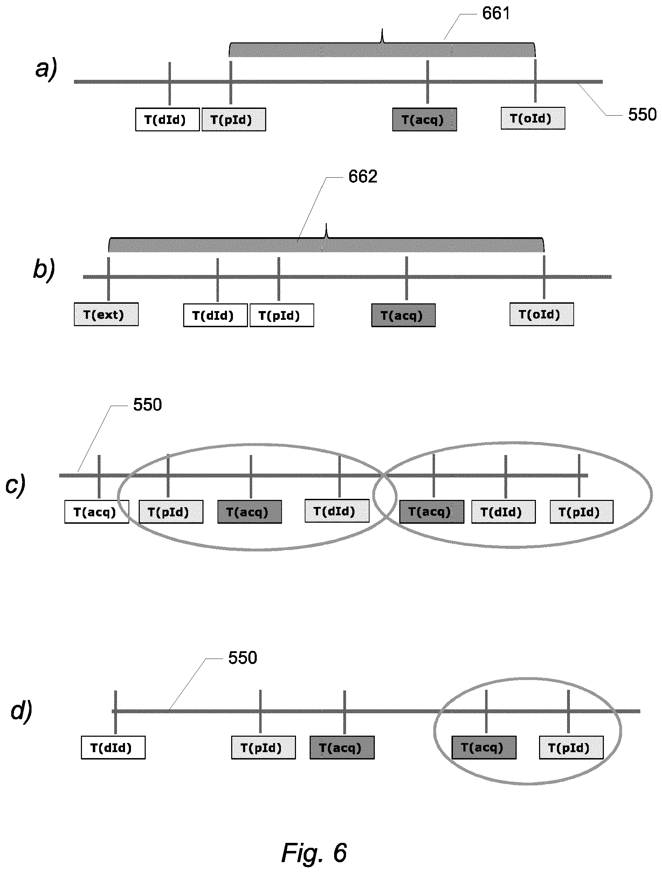

In the following, alternative methods of associating the measurement with a patient ID based on the registered identifiers and times will be described with reference to figs. 5 and 6 below.

Still referring to fig. 4, in some embodiments, when connected to the host system 401, the following information is included in all messages from the electronic devices 403A and 403B, respectively:
1) Device ID
2) Current transmission time - e.g. the time as it is setup on the instrument.

If there is a difference between the current transmission time as received by the host system and the current time determined by the host system, the host system may try to automatically adjust the device time of the electronic device (if such functionality is supported by the electronic device) and/or save the time difference in the database. Due to the above comparison of the device time and the time determined by the host system, any time a message is received from that electronic device, the host system can determine the exact time of measurement.

Some electronic devices may use only hour and minutes as timestamp and since the time required by each measurement may be shorter (some devices may allow 1-3 measurements per minute) it is possible that two measurements can have the same timestamp. Upon registration of an electronic device by the registration device, the registration device may retrieve information about such limitations of the electronic device, and the registration device may inform the user that there is a potential risk of not automatically being able to identify a patient sample with the registrations performed if two registrations are performed within 60 seconds. In case a user disregards the warning and performs two measurements with same timestamp, the host system may later query for either performing a sample rerun or that the user manually maps the patient ID with the measurement. Generally, embodiments of the system described herein may detect situations that involve a risk of incorrect associations, e.g. due to limited time resolution of the timers, and perform an appropriate action so as to avoid any inadvertent associations.

Figs. 5 and 6 illustrate different embodiments of a method of acquiring patient-related data. In particular, figs. 5a-d and figs. 6 a-d each illustrate a time line 550, and the sequential order of different registration events. In the following, the following notation will be used:
*T(pld*) = Time stamp of patient identification by the registration device
*T(dld*) = Time stamp of the electronic device identification by the registration device
*T(old*) = Time stamp of the operator identification by the registration device
*T(acq)* = Time stamp of the data acquisition (measurement) performed by the electronic device
*T(ext)* = Time stamp of an external trigger event
*T(par)* = Time stamp of a computerized parameter
Δ*T(x,y)* = Time interval between two time stamps y and x (where y is an older event than x), e.g. Δ*T(pld,dld*) = Time interval between *T(dld*) and *T(pld*)

For the purpose of the following description, it is assumed that the identifiers are in the form of machine readable codes that can be scanned by the operator using the registration device, and that the patient-related data relates to measurements. However, it will be appreciated that any other form of obtaining the identifiers may be used, and that the patient-related data may be indicative of data other than measurements. Furthermore, in the following examples, some assumptions under which the host system performs the association between measurements and patient identifiers are specified. During operation of the system, compliance with these assumptions may e.g. be ensured by suitable standard operating procedures instructing the operators about how to operate the system.

Generally the process of associating the received patient-related data with a patient identifier may initially determine one or more received sets of identification data items where each set comprises a device identification data item that matches the obtained device identifier of the electronic device from which the patient-related data has been received. The process may then associate the received patient-related data with the patient identification data item of one of the determined sets of identification data items based on at least the received one or more identification time stamps and the received acquisition time stamp as described in greater detail below.

In the example of fig. 5a the host system determines a registration time interval based on an assumption that the operator scans the electronic device ID, the operator ID, and the patient ID, and subsequently performs the measurement using the electronic device. After receipt of the three identifiers, the registration device blocks the registration device to be used for a new session (i.e. further scans of identifiers) until a predetermined time interval (e.g. of 3 minutes) has elapsed starting from the receipt of the last of the three scans. In fig. 5a, the end of the time-out period is labeled by the time *T(par),* e.g. as determined by expiry of a timer of the registration device. The host system subsequently determines the time interval Δ*T(par, pld)* (designated by reference numeral 551) between the scanning of the patient ID and the expiry of the timer *T(par).* The host system then identifies a measurement originating from the electronic device with device ID *dld* and where the measurement has a time stamp *T(acq)* within the time interval Δ*T(par, pld).* Alternatively, the host system may determine the time interval as the time starting from the earliest of the group of three scans and ending at *T(par).* The group of scans including the operator ID, device ID and patient ID relating to the same registration event may be determined by the host system as a group received within the same message from the registration device, or as a group determined by the host system from the overall sequence and/or relative timing of received identifiers.

The example of fig. 5b is similar to that of fig. 5a. However, in this example the acquisition time interval 552 is not triggered by one of the scans but by a predetermined external event at time *T(ext),* e.g. by the operator pressing a predetermined button (or performing another predetermined action) on the registration device. For example, the operator may scan the three identifiers and then press a "start" button which causes the registration device to forward a message to the host system including the three scanned identifiers and a single time stamp *T(ext)* indicative of the time at which the user has pressed the "start" button. Furthermore, the registration device may block the system for a predetermined time interval until *T(par)* so as to prevent scanning of further identifiers during the time interval for measurement. The host system subsequently determines the time interval Δ*T(par, ext)* (designated by reference numeral 552) between the external event and the expiry of the timer *T(par).* The host system then identifies a measurement originating from the electronic device with device ID *dld* and where the measurement has a time stamp *T(acq)* within the time interval Δ*T(par, ext).*

The example of fig. 5c is similar to that of fig. 5a but where the acquisition time interval extends to both sides of the scan that triggers the time interval. In the example of fig. 5c the host system determines a registration time interval based on an assumption that the operator scans the electronic device ID, the operator ID, and the patient ID, and performs the measurement using the electronic device within a predetermined time interval after or prior to the scanning of the identifiers. To this end, after receipt of the three identifiers, the registration device blocks the registration device to be used for a new session (i.e. further scans of identifiers) until a predetermined time interval (e.g. of 3 minutes) has elapsed starting from the receipt of the last of the three scans.

The host system subsequently determines the time interval 553 to extend a predetermined period prior to the last one of the three scans and a predetermined period after the last scan, where the length of each predetermined period may be selected to be equal to the length of the blocking period. Hence, the system assumes (or checks based on data received from the registration device) that the system was not blocked during the predetermined period prior to the last scan. The host system then identifies a measurement originating from the electronic device with device ID *dld* and where the measurement has a time stamp *T(acq)* within the time interval 553. In one embodiment, if multiple such measurements are identified, only the most recent one is considered. Alternatively, the system may cause a rerun of the measurement process, e.g. after issuing an error message.

The example of fig. 5d is similar to that of fig. 5c in that the time interval 554 extends to both sides of the triggering event. However, in this example, the time interval 554 is triggered by an external event (e.g. by a user pressing a given button on the registration device) rather than by one of the scans, i.e. similar to the triggering described in the example of fig. 5b.

In the example of fig. 6a the host system determines a registration time interval based on an assumption that the operator initially scans the device ID of the electronic device, and the patient ID, then performs the measurement using the electronic device, and finally scans the operator ID. This scenario is similar to the example described with reference to fig. 4 above. In particular, the host system determines the time interval Δ*T(old, pld)* (designated by reference numeral 661) between the scanning of the patient ID and the scanning of the operator ID. The host system then identifies a measurement originating from the electronic device with device ID *dld* and where the measurement has a time stamp *T(acq)* within the time interval Δ*T(old, pld*)*.* Alternatively, the host system may determine the time interval as the time from the earliest to the latest of a group of three scans of the operator ID, device ID and patient ID relating to the same registration event (e.g. as a group received from the registration device, or as a group determined by the host system from the overall sequence and/or relative timing of received identifiers).

In the example of fig. 6b, the measuring process is assumed initiated by an external event, e.g. the receipt by the registration device (e.g. via the host system) of a test order from a Hospital Information System (HIS). In response to the external event, the operator is assumed to scan the device ID and the patient ID. Then the operator is assumed to perform the measurement using the electronic device, and finally to scan the operator ID. In this example the host system determines the time interval 662 between the time *T(ext)* at which the external event has occurred, and the latest of the three expected scans of identifiers, in this case the scan of the operator ID. The host system then identifies a measurement originating from the electronic device with device ID *dld* and where the measurement has a time stamp *T(acq)* within the time interval Δ*T(old, ext*)*.*

In the example of fig. 6c, the host system does not receive any operator ID from the registration device, but merely a patient ID and a device ID as well as their associate time stamps. The operator is thus assumed to scan the patient ID and the device ID, and to perform a measurement. These actions may be performed in any order; however, the subsequent association is based on the assumption that each registration/measuring event comprises at least a patient ID, a device ID, and a measurement. In this example the host system associates the received measurements with respective patient IDs based on a sequential grouping of the registration and measuring events. The host system may, based on the sequential order of the corresponding time stamps, iteratively identify groups, each group comprising a patient ID, a device ID and a measurement, as illustrated in fig. 6c. For example, in each iteration, the host system may identify the earliest time stamp that has not yet been assigned to a group, and assign this time stamp to a new group. Subsequently, the host system may identify the earliest time stamps that are subsequent to the initially identified time stamp, and add them to the new group until the group consists of a time stamp associated with a patient ID, a time stamp associated with a device ID, and a time stamp associated with a measurement. It will be appreciated that this embodiment may be modified to also comprise operator IDs. In such an embodiment, each group is determined, based on their respective time stamps, as a group comprising a patient ID, a device ID, an operator ID, and a measurement.

The example of fig. 6d is similar to fig. 6c, in that the association of measurements to patient devices is based on a determination of groups of registration/measurement events based on the respective time stamps. However, in this embodiment, the grouping is only based on the time stamps of the registered patient IDs and the time stamps of the measurements. The operator is assumed to only register the device ID once (e.g. in the morning when the operator starts working with the corresponding electronic device, or every time the operator switches to a new electronic device). Subsequently, the operator only needs to scan the patient ID and perform a measurement with the previously registered electronic device. In this example the host system associates the received measurements with corresponding patient IDs based on the initially registered device ID, and based on a sequential grouping of the registration and measuring events. The host system may, based on the sequential order of the corresponding time stamps, iteratively identify groups, each group comprising a patient ID and a measurement, as illustrated in fig. 6d. It will be appreciated that this embodiment may be modified to also comprise operator IDs. In such an embodiment, the operator may perform an initial registration of both the device ID and the operator ID, thus allowing the registration device to perform a compliance test. Subsequently, the operator merely performs registrations of the respective patient IDs and performs measurements. It will further be appreciated that also some of the other examples described above may be modified in that the device ID and/or the operator ID does not need to be registered in connection with every measurement, but only during an initial start-up and/or upon a change in operator and/or electronic device. For example, in the examples of figs. 5a through 5d, as well as 6a and 6b, the definition of time intervals may be based on the patient ID and/or an external event.

Hence the above examples illustrate that a unique identification of an acquisition time stamp *T(acq)* to be associated with a patient identification may be based on one or more time stamps associated with the registration of one or more identifiers and on the patient identifier and the device identifier. Generally, in a first group of embodiments, the association is based on an identification of a time interval Δ*T(x,y)* in order to constrain *T(acq)* inside the boundaries of the interval, i.e. identify the correct acquisition time stamp as the one that falls within the identified time interval. In a second group of embodiments the association is based on an identification of a group of actions and an association of the identified group with *T(acq).* The identification of the group and the linking of *T(acq)* to the group may be based on the sequential order of the actions and/or their relative timing, and on the relative timing of *T(acq)* relative to the members of the identified group. The group may include at least the registration of the patient ID.

It will be appreciated that a system implementing an embodiment of the method described herein may be configured to implement one or more embodiments, e.g. including one or more embodiments described above. In particular, such a system may be adapted to allow an operator to select one of a set of available embodiments. For example such a selection may be performed during an initial configuration of the system, thus allowing adaptation of the system to the capabilities of the devices available at a given healthcare facility and/or to existing standard operating procedures at that healthcare facility. In particular, the system may allow an operator to select a mode of operation from a plurality of available modes of operation. A mode of operation may e.g. be identified by the association criteria to be used (e.g. based on time intervals or on the sequential order of trigger events, etc.) and by an identification of which types of time stamps or other triggers to be used during that association.

For example, in order to configure such a configurable system to implement the embodiment described in connection with fig. 5a, an operator may select an association based on time intervals from a list of types of association criteria. In a subsequent step, the operator may select receipt of a patient ID time stamp as a start trigger for a time interval, and the expiry of a timer (e.g. of configurable duration) as a termination trigger for the time interval. The selection of the trigger may be performed as a selection from a list of available triggers. A list of association criteria may e.g. include "one-sided time interval with start trigger", "two-sided time interval with central trigger", "identification of groups of events", etc. A list of available start or termination triggers may e.g. include "patient ID time stamp", "device ID time stamp", "operator ID time stamp", "external event", "timer", and/or the like.

It will be appreciated that such a configuration may be performed in a variety of different ways, e.g. at run-time during an initial configuration or set-up stage where an operator may be presented with a sequence of user interface elements allowing the operator to select the configurable modes of operation. Alternatively, the configuration may be performed prior to the initial operation of the system, e.g. by means of creating a configuration file that includes the relevant configuration parameters, or even at compile time of a corresponding software application.

Although some embodiments have been described and shown in detail, the invention is not restricted to them, but may also be embodied in other ways within the scope of the subject matter defined in the following claims.

The method, product means, system, and device described herein can be implemented by means of hardware comprising several distinct elements, and/or partly or completely by means of a suitably programmed microprocessor. In the device claims enumerating several means, several of these means can be embodied by one and the same item of hardware, e.g. a suitably programmed microprocessor, one or more digital signal processor, or the like. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, integers, steps or components but does not preclude the presence or addition of one or more other features, integers, steps, components or groups thereof.

## Claims

1. A method of registering patient related data performed by a system comprising a data processing system (101), a registration device (102), and an electronic device (103), wherein the data processing system is configured to operate in one of a plurality of available modes of operation, the method comprising:
the registration device (102) receiving (S301, S304) a set of identifiers each identifying a respective object;
for each received identifier, the registration device (102) communicating (S305) an identification data item to the data processing system (101) via a first communications interface, the identification data item comprising the identifier and an identification time stamp indicative of an identification time at which the object has been identified;
after receipt of the set of identifiers, the registration device (102) preventing (S306), for a predetermined data acquisition time interval (551, 552, 553, 554, 661, 662), at least communicating further identification data items including identifiers different from said set of received identifiers;
the method further comprising, by the data processing system (101):
- receiving (S311), from the registration device (102) via the first communications interface, a set of the identification data items from a data input unit of the registration device and the identification time stamps from a timer circuit of the registration device, each identification time stamp being associated with at least one of the identification data items by the registration device, wherein the set of identification data items comprises a device identification data item (dld) identifying an electronic device and a patient identification data item (pld) identifying a patient;
- receiving, from the electronic device (103) via a second communications interface, patient-related data (PD) to be registered from a patient data acquisition device (219) comprised in the electronic device and an acquisition time stamp (T(acq)) indicative of an acquisition time at which the patient-related data has been acquired, wherein the acquisition time stamp is obtained from a timer circuit of the electronic device, wherein receiving the patient-related data (PD) to be registered and the acquisition time stamp (T(acq)) further comprises obtaining a device identifier (dld) of the electronic device (103) from which the patient-related data is received; and
- associating (S313), by the data processing system, the received patient-related data with an identified patient based on at least the received device identification data item, the received patient identification data item, the received one or more identification time stamps and the received acquisition time stamp;
wherein associating comprises comparing the acquisition time with a first time identified by a first one of the one or more identification time stamps, and wherein comparing comprises determining whether the acquisition time lies within the predetermined data acquisition time interval (551, 552, 553, 554, 661, 662) determined from at least said first identification time stamp, and associating further comprises associating, if the acquisition time lies within said predetermined data acquisition time interval, the patient-related data with the patient identification data item, and wherein associating the received patient-related data with the identified patient is further based on said obtained device identifier of the electronic device from which the patient-related data is received; and
wherein the set of identification data items and the one or more identification time stamps from the registration device is received by the data processing system asynchronously with respect to receiving the patient-related data and the acquisition time stamp from the electronic device.

2. A method according to claim 1, wherein the available modes of operation are identified by association criteria to be used, wherein the association criteria are based on time intervals or on the sequential order of trigger events, and by identification of type of time stamps or other triggers used during association, wherein the other triggers comprise a predetermined operator input to the registration device (102), the receipt of a message by the registration device from an external system, or the expiry of a timer of the registration device.

3. A method according to claim 1, wherein the available modes of operation are configurable.

4. A method according to claim 1, wherein the data acquisition time interval is a time interval starting at said first time and having a predetermined length.

5. A method according to claim 1, wherein the data acquisition time interval extends a first predetermined period prior to the first time and a second predetermined period after the first time.

6. A method according to claim 1, wherein receiving a set of identification data items and one or more identification time stamps comprises receiving at least said first and a second identification time stamp, the second identification time stamp identifying a second time; and wherein the data acquisition time interval is a time interval starting at said first time and ending at said second time.

7. A method according to claim 6, wherein the second time is an identification time at which one of the identification data items has been obtained.

8. A method according to claim 6, wherein the second time is a time at which a predetermined trigger event has occurred.

9. A method according to any one of claims 1 through 8, wherein the first time is a time at which a predetermined trigger event has occurred.

10. A method according to any one of claims 1 through 8, wherein the first time is an identification time at which one of the identification data items has been obtained.

11. A method according to any one of claims 1 through 10, wherein receiving the set of identification data items comprises receiving a plurality of identification data items and determining said set of identification data items from said received plurality of received identification data items as a set of identification data items associated with said first identification time stamp.

12. A method according to claim 11, wherein determining said set of identification data items comprises determining a set of obtained identification data items from at least one of a sequential order in which the identification data items have been obtained and a relative timing of respective identification times at which the respective identification data items have been obtained.

13. A method according to any one of the preceding claims, wherein each of the one or more identification time stamps is indicative of an identification time at which a respective one of the identification data items has been obtained; and wherein associating comprises comparing a sequence of identification time stamps and a sequence of data acquisition time stamps, and identifying associated sets of identification events and data acquisition events from said comparison.

14. A method according to claim 13, wherein the identification time stamps comprise a patient identification time stamp (T(pld)) and a device identification time stamp (T(dld)), the patient identification time stamp being indicative of a patient identification time at which the patient identification data item (pld) has been obtained, and the device identification time stamp being indicative of a device identification time at which the device identification data item (did) has been obtained; and wherein identifying associated sets of identification events and data acquisition events comprises identifying triplets of respective patient identification time stamps, device identification time stamps, and data acquisition time stamps (T(acq)).

15. A method according to claim 13, wherein the identification time stamps comprise a patient identification time stamp (T(pld)) indicative of a patient identification time at which the patient identification data item (pld) has been obtained; and
wherein identifying associated sets of identification events and data acquisition events comprises identifying duplets of respective patient identification time stamps and data acquisition time stamps (T(acq)).

16. A method according to any one of the preceding claims, wherein the set of identification data items further comprises an operator identification data item (old) identifying an operator of the electronic device.

17. A method according to any one of the preceding claims, wherein receiving an acquisition time stamp (T(acq)) from the electronic device (103) via the second communication interface further comprises receiving, from the electronic device, a control time stamp (TED(trans)) indicative of a current time determined by the electronic device; and correcting (S312) the acquisition time stamp based on a comparison of the control time stamp with a current time determined by the data processing system.

18. A system configured to perform the method for registering patient-related data according to claim 1, the system comprising the registration device (102), the electronic device (103), and the data processing system (101), wherein the registration device comprises input means for receiving the set of identifiers, and the first communications interface adapted to forward the identification data item indicative of the received identifier to the data processing system;
wherein the electronic device comprises means for acquiring the patient related data and the second communications interface adapted to communicate the acquired patient-related data to the data processing system.

19. A computer program product comprising program code means adapted to cause the system of claim 18 to perform the method according to claim 1, when said program code means are executed by the system.

## Patentansprüche

1. Verfahren zum Registrieren patientenbezogener Daten, durchgeführt von einem System, das ein Datenverarbeitungssystem (101), eine Registrierungsvorrichtung (102) und eine elektronische Vorrichtung (103) umfasst, wobei das Datenverarbeitungssystem ausgebildet ist, in einem von einer Vielzahl von verfügbaren Betriebsarten zu arbeiten, wobei das Verfahren umfasst:
die Registrierungsvorrichtung (102), die einen Satz von Identifikatoren empfängt (S301, S304), von denen jeder ein jeweiliges Objekt identifiziert;
für jeden empfangenen Identifikator übermittelt (S305) die Registrierungsvorrichtung (102) ein Identifikationsdatenelement über eine erste Kommunikationsschnittstelle an das Datenverarbeitungssystem (101), wobei das Identifikationsdatenelement den Identifikator und einen Identifikationszeitstempel umfasst, der eine Identifikationszeit angibt, zu der das Objekt identifiziert wurde;
nach Empfang des Satzes von Identifikatoren verhindert (S306) die Registrierungsvorrichtung (102) für ein vorbestimmtes Datenerfassungszeitintervall (551, 552, 553, 554, 661, 662) mindestens, dass weitere Identifikationsdatenelemente übermittelt werden, die Identifikatoren einschließen, die sich von dem Satz empfangener Identifikatoren unterscheiden;
das Verfahren weiter umfassend, durch das Datenverarbeitungssystem (101):
- Empfangen (S311), von der Registrierungsvorrichtung (102) über die erste Kommunikationsschnittstelle, eines Satzes von Identifikationsdatenelementen aus einer Dateneingabeeinheit der Registrierungsvorrichtung und des Identifikationszeitstempels aus einer Zeitgeberschaltung der Registrierungsvorrichtung, wobei jeder Identifikationszeitstempel von der Registrierungsvorrichtung mindestens einem der Identifikationsdatenelemente zugeordnet wird, wobei der Satz von Identifikationsdatenelementen ein Vorrichtungsidentifikationsdatenelement (did), das eine elektronische Vorrichtung identifiziert, und ein Patientenidentifikationsdatenelement (pld), das einen Patienten identifiziert, umfasst;
- Empfangen, von der elektronischen Vorrichtung (103) über eine zweite Kommunikationsschnittstelle, von patientenbezogenen Daten (PD), die von einer Patientendatenerfassungsvorrichtung (219), die in der elektronischen Vorrichtung enthalten ist, registriert werden sollen, sowie eines Erfassungszeitstempels (T(acq)), der eine Erfassungszeit angibt, zu der die patientenbezogenen Daten erfasst wurden, wobei der Erfassungszeitstempel von einer Zeitgeberschaltung der elektronischen Vorrichtung erhalten wird, wobei das Empfangen der zu registrierenden patientenbezogenen Daten (PD) und des Erfassungszeitstempels (T(acq)) weiter das Erhalten eines Vorrichtungsidentifikators (did) der elektronischen Vorrichtung (103), von der die patientenbezogenen Daten empfangen werden, umfasst; und
- Zuordnen (S313), durch das Datenverarbeitungssystem, der empfangenen patientenbezogenen Daten zu einem identifizierten Patienten auf der Grundlage mindestens des empfangenen Vorrichtungsidentifikationsdatenelements, des empfangenen Patientenidentifikationsdatenelements, des empfangenen einen oder der empfangenen mehreren Identifikationszeitstempel und des empfangenen Erfassungszeitstempels;
wobei das Zuordnen das Vergleichen der Erfassungszeit mit einer ersten Zeit umfasst, die durch einen ersten des einen oder der mehreren Identifikationszeitstempel identifiziert wird, und wobei das Vergleichen das Bestimmen umfasst, ob die Erfassungszeit innerhalb des vorbestimmten Datenerfassungszeitintervalls (551, 552, 553, 554, 661, 662) liegt, das anhand mindestens des ersten Identifikationszeitstempels bestimmt wird, und das Zuordnen weiter das Zuordnen der patientenbezogenen Daten zu dem Patientenidentifikationsdatenelement umfasst, falls die Erfassungszeit innerhalb des vorbestimmten Datenerfassungszeitintervalls liegt, und wobei das Zuordnen der empfangenen patientenbezogenen Daten zu dem identifizierten Patienten weiter auf dem erhaltenen Vorrichtungsidentifikator der elektronischen Vorrichtung basiert, von der die patientenbezogenen Daten empfangen werden; und
wobei der Satz von Identifikationsdatenelementen und der eine oder die mehreren Identifikationszeitstempel von der Registrierungsvorrichtung von dem Datenverarbeitungssystem asynchron bezüglich des Empfangens der patientenbezogenen Daten und des Erfassungszeitstempels von der elektronischen Vorrichtung empfangen werden.

2. Verfahren nach Anspruch 1, wobei die verfügbaren Betriebsarten durch Zuordnungskriterien zur Verwendung, wobei die Zuordnungskriterien auf der Grundlage von Zeitintervallen oder der sequenziellen Reihenfolge von Auslöserereignissen basieren, und durch Identifikation der Art der Zeitstempel oder anderer während der Zuordnung verwendeter Auslöser identifiziert werden, wobei die anderen Auslöser eine vorbestimmte Bedienereingabe an die Registrierungsvorrichtung (102), den Empfang einer Nachricht durch die Registrierungsvorrichtung von einem externen System oder das Ablaufen eines Zeitgebers der Registrierungsvorrichtung umfassen.

3. Verfahren nach Anspruch 1, wobei die verfügbaren Betriebsarten konfigurierbar sind.

4. Verfahren nach Anspruch 1, wobei das Datenerfassungszeitintervall ein Zeitintervall ist, das zu der ersten Zeit beginnt und eine vorbestimmte Länge aufweist.

5. Verfahren nach Anspruch 1, wobei das Datenerfassungszeitintervall eine erste vorbestimmte Zeitdauer vor der ersten Zeit und eine zweite vorbestimmte Zeitdauer nach der ersten Zeit umfasst.

6. Verfahren nach Anspruch 1, wobei das Empfangen eines Satzes von Identifikationsdatenelementen und eines oder mehrerer Identifikationszeitstempel das Empfangen mindestens des ersten und eines zweiten Identifikationszeitstempels umfasst, wobei der zweite Identifikationszeitstempel eine zweite Zeit identifiziert; und wobei das Datenerfassungszeitintervall ein Zeitintervall ist, das bei der ersten Zeit beginnt und bei der zweiten Zeit endet.

7. Verfahren nach Anspruch 6, wobei die zweite Zeit eine Identifikationszeit ist, zu der eines der Identifikationsdatenelemente erhalten wurde.

8. Verfahren nach Anspruch 6, wobei die zweite Zeit ein Zeitpunkt ist, zu dem ein vorbestimmtes Auslöseereignis aufgetreten ist.

9. Verfahren nach einem der Ansprüche 1 bis 8, wobei die erste Zeit ein Zeitpunkt ist, zu dem ein vorbestimmtes Auslöseereignis aufgetreten ist.

10. Verfahren nach einem der Ansprüche 1 bis 8, wobei die erste Zeit eine Identifikationszeit ist, zu der eines der Identifikationsdatenelemente erhalten wurde.

11. Verfahren nach einem der Ansprüche 1 bis 10, wobei das Empfangen des Satzes von Identifikationsdatenelementen das Empfangen einer Vielzahl von Identifikationsdatenelementen und das Bestimmen des Satzes von Identifikationsdatenelementen aus der empfangenen Vielzahl von empfangenen Identifikationsdatenelementen als ein Satz von Identifikationsdatenelementen, der dem ersten Identifikationszeitstempel zugeordnet ist, umfasst.

12. Verfahren nach Anspruch 11, wobei das Bestimmen des Satzes von Identifikationsdatenelementen das Bestimmen eines Satzes von erhaltenen Identifikationsdatenelementen aus mindestens einem von einer sequenziellen Reihenfolge, in der die Identifikationsdatenelemente erhalten wurden, und einem relativen Zeitpunkt der jeweiligen Identifikationszeiten, zu denen die jeweiligen Identifikationsdatenelemente erhalten wurden.

13. Verfahren nach einem der vorstehenden Ansprüche, wobei jeder des einen oder der mehreren Identifikationszeitstempel einen Identifikationszeitpunkt angibt, zu dem jeweils eines der Identifikationsdatenelemente erhalten wurde; und
wobei das Zuordnen das Vergleichen einer Sequenz von Identifikationszeitstempeln und einer Sequenz von Datenerfassungszeitstempeln sowie das Identifizieren zugeordneter Sätze von Identifikationsereignissen und Datenerfassungereignissen aus dem Vergleich umfasst.

14. Verfahren nach Anspruch 13, wobei die Identifikationszeitstempel einen Patientenidentifikationszeitstempel (T(pld)) und einen Vorrichtungsidentifikationszeitstempel (T(dld)) umfassen, wobei der Patientenidentifikationszeitstempel eine Patientenidentifikationszeit angibt, zu der das Patientenidentifikationsdatenelement (pld) erhalten wurde, und wobei der Vorrichtungsidentifikationszeitstempel einen Vorrichtungsidentifikationszeitpunkt angibt, zu dem das Vorrichtungsidentifikationsdatenelement (did) erhalten wurde; und wobei das Identifizieren zugeordneter Sätze von Identifikationsereignissen und Datenerfassungereignissen das Identifizieren von Dreiergruppen aus jeweiligen Patientenidentifikationszeitstempeln, Vorrichtungsidentifikationszeitstempeln und Datenerfassungszeitstempeln (T(acq)) umfasst.

15. Verfahren nach Anspruch 13, wobei die Identifikationszeitstempel einen Patientenidentifikationszeitstempel (T(pld)) umfassen, der eine Patientenidentifikationszeit angibt, zu der das Patientenidentifikationsdatenelement (pld) erhalten wurde; und
wobei das Identifizieren zugeordneter Sätze von Identifikationsereignissen und Datenerfassungereignissen das Identifizieren von Zweiergruppen aus den jeweiligen Patientenidentifikationszeitstempeln und Datenerfassungszeitstempeln (T(acq)) umfasst.

16. Verfahren nach einem der vorstehenden Ansprüche, wobei der Satz von Identifikationsdatenelementen weiter ein Bedieneridentifikationsdatenelement (old) umfasst, das einen Bediener der elektronischen Vorrichtung identifiziert.

17. Verfahren nach einem der vorstehenden Ansprüche, wobei das Empfangen eines Erfassungszeitstempels (T(acq)) von der elektronischen Vorrichtung (103) über die zweite Kommunikationsschnittstelle weiter das Empfangen, von der elektronischen Vorrichtung, eines Steuerzeitstempels (TED(trans)), der eine von der elektronischen Vorrichtung bestimmte aktuelle Zeit angibt; und das Korrigieren (S312) des Erfassungszeitstempels auf der Grundlage eines Vergleichs des Steuerzeitstempels mit einer von dem Datenverarbeitungssystem bestimmten aktuellen Zeit umfasst.

18. System, das ausgebildet ist, das Verfahren zum Registrieren patientenbezogener Daten nach Anspruch 1 durchzuführen, wobei das System die Registrierungsvorrichtung (102), die elektronische Vorrichtung (103) und das Datenverarbeitungssystem (101) umfasst, wobei die Registrierungsvorrichtung Eingabemittel zum Empfangen des Satzes von Identifikatoren sowie die erste Kommunikationsschnittstelle umfasst, die dazu angepasst ist, das Identifikationsdatenelement, das den empfangenen Identifikator angibt, an das Datenverarbeitungssystem weiterzuleiten;
wobei die elektronische Vorrichtung Mittel zum Erfassen der patientenbezogenen Daten sowie die zweite Kommunikationsschnittstelle umfasst, die angepasst ist, die erfassten patientenbezogenen Daten an das Datenverarbeitungssystem zu übermitteln.

19. Computerprogrammprodukt, umfassend Programmcodemittel, die angepasst sind, um das System nach Anspruch 18 dazu zu veranlassen, das Verfahren nach Anspruch 1 durchzuführen, wenn die Programmcodemittel von dem System ausgeführt werden.

## Revendications

1. Procédé d'enregistrement de données relatives au patient réalisé par un système comprenant un système (101) de traitement de données, un dispositif d'enregistrement (102) et un dispositif électronique (103), dans lequel le système de traitement de données est configuré pour fonctionner dans l'un d'une pluralité de modes de fonctionnement disponibles, le procédé comprenant :
la réception (S301, S304), par le dispositif d'enregistrement (102), d'un ensemble d'identifiants identifiant chacun un objet respectif ;
pour chaque identifiant reçu, la communication (S305), par le dispositif d'enregistrement (102), d'un élément de données d'identification au système de traitement de données (101) via une première interface de communication, l'élément de données d'identification comprenant l'identifiant et un horodatage d'identification indicatif d'un temps d'identification auquel l'objet a été identifié ;
après réception de l'ensemble d'identifiants, le fait d'empêcher (S306), par le dispositif d'enregistrement (102), pendant un intervalle de temps (551, 552, 553, 554, 661, 662) d'acquisition de données prédéterminé, au moins la communication d'autres éléments de données d'identification incluant des identifiants différents dudit ensemble d'identifiants reçus ;
le procédé comprenant en outre, par le système de traitement de données (101) :
- la réception (S311), à partir du dispositif d'enregistrement (102) via la première interface de communication, d'un ensemble des éléments de données d'identification à partir d'une unité d'entrée de données du dispositif d'enregistrement et des horodatages d'identification à partir d'un circuit de temporisateur du dispositif d'enregistrement, chaque horodatage d'identification étant associé à au moins un des éléments de données d'identification par le dispositif d'enregistrement, dans lequel l'ensemble d'éléments de données d'identification comprend un élément de données d'identification de dispositif (dld) identifiant un dispositif électronique et un élément de données d'identification de patient (pld) identifiant un patient ;
- la réception, à partir du dispositif électronique (103) via une deuxième interface de communication, de données relatives au patient (PD) à enregistrer à partir d'un dispositif (219) d'acquisition de données de patient compris dans le dispositif électronique et d'un horodatage d'acquisition (T(acq)) indicatif d'un temps d'acquisition auquel les données relatives au patient ont été acquises, dans lequel l'horodatage d'acquisition est obtenu à partir d'un circuit de temporisateur du dispositif électronique, dans lequel la réception des données relatives au patient (PD) à enregistrer et l'horodatage d'acquisition (T(acq)) comprend en outre l'obtention d'un identifiant de dispositif (dld) du dispositif électronique (103) à partir duquel les données relatives au patient sont reçues ; et
- l'association (S313), par le système de traitement de données, des données relatives au patient reçues à un patient identifié sur la base d'au moins l'élément de données d'identification de dispositif reçu, de l'élément de données d'identification de patient reçu, des un ou plusieurs horodatages d'identification reçus et de l'horodatage d'acquisition reçu ;
dans lequel l'association comprend la comparaison du temps d'acquisition avec un premier temps identifié par un premier parmi les un ou plusieurs horodatages d'identification et dans lequel la comparaison comprend le fait de déterminer si le temps d'acquisition se situe dans l'intervalle de temps (551, 552, 553, 554, 661, 662) d'acquisition de données prédéterminé déterminé à partir de l'au moins ledit premier horodatage d'identification, et l'association comprend en outre l'association, si le temps d'acquisition se situe dans ledit intervalle de temps d'acquisition de données prédéterminé, des données relatives au patient avec l'élément de données d'identification de patient et dans lequel l'association des données relatives au patient reçues avec le patient identifié est en outre basée sur ledit identifiant de dispositif obtenu du dispositif électronique à partir duquel les données relatives au patient sont reçues ; et
dans lequel l'ensemble d'éléments de données d'identification et les un ou plusieurs horodatages d'identification provenant du dispositif d'enregistrement sont reçus par le système de traitement de données de manière asynchrone par rapport à la réception des données relatives au patient et de l'horodatage d'acquisition provenant du dispositif électronique.

2. Procédé selon la revendication 1, dans lequel les modes de fonctionnement disponibles sont identifiés par des critères d'association devant être utilisés, dans lequel les critères d'association sont basés sur des intervalles de temps ou sur l'ordre séquentiel d'événements déclencheurs, et par l'identification du type d'horodatages ou d'autres déclencheurs utilisés pendant l'association, dans lequel les autres déclencheurs comprennent une entrée opérateur prédéterminée vers le dispositif d'enregistrement (102), la réception d'un message par le dispositif d'enregistrement à partir d'un système externe, ou l'expiration d'un temporisateur du dispositif d'enregistrement.

3. Procédé selon la revendication 1, dans lequel les modes de fonctionnement disponibles sont configurables.

4. Procédé selon la revendication 1, dans lequel l'intervalle de temps d'acquisition de données est un intervalle de temps commençant audit premier temps et présentant une longueur prédéterminée.

5. Procédé selon la revendication 1, dans lequel l'intervalle de temps d'acquisition de données s'étend sur une première période prédéterminée avant le premier temps et une deuxième période prédéterminée après le premier temps.

6. Procédé selon la revendication 1, dans lequel la réception d'un ensemble d'éléments de données d'identification et d'un ou plusieurs horodatages d'identification comprend la réception d'au moins ledit premier et un deuxième horodatage d'identification, le deuxième horodatage d'identification identifiant un deuxième temps ; et dans lequel l'intervalle de temps d'acquisition de données est un intervalle de temps commençant audit premier temps et se terminant audit deuxième temps.

7. Procédé selon la revendication 6, dans lequel le deuxième temps est un temps d'identification auquel l'un des éléments de données d'identification a été obtenu.

8. Procédé selon la revendication 6, dans lequel le deuxième temps est un temps auquel un événement déclencheur prédéterminé est apparu.

9. Procédé selon l'une quelconque des revendications 1 à 8, dans lequel le premier temps est un temps auquel un événement déclencheur prédéterminé est apparu.

10. Procédé selon l'une quelconque des revendications 1 à 8, dans lequel le premier temps est un temps d'identification auquel l'un des éléments de données d'identification a été obtenu.

11. Procédé selon l'une quelconque des revendications 1 à 10, dans lequel la réception de l'ensemble d'éléments de données d'identification comprend la réception d'une pluralité d'éléments de données d'identification et la détermination dudit ensemble d'éléments de données d'identification à partir de ladite pluralité reçue d'éléments de données d'identification reçus en tant qu'ensemble d'éléments de données d'identification associés audit premier horodatage d'identification.

12. Procédé selon la revendication 11, dans lequel la détermination dudit ensemble d'éléments de données d'identification comprend la détermination d'un ensemble d'éléments de données d'identification obtenus à partir d'au moins l'un parmi un ordre séquentiel dans lequel les éléments de données d'identification ont été obtenus et un séquencement relatif des temps d'identification respectifs auxquels les éléments de données d'identification respectifs ont été obtenus.

13. Procédé selon l'une quelconque des revendications précédentes, dans lequel chacun des un ou plusieurs horodatages d'identification est indicatif d'un temps d'identification auquel l'un respectif des éléments de données d'identification a été obtenu ; et
dans lequel l'association comprend la comparaison d'une séquence d'horodatages d'identification et d'une séquence d'horodatages d'acquisition de données, et l'identification d'ensembles associés d'événements d'identification et d'événements d'acquisition de données à partir de ladite comparaison.

14. Procédé selon la revendication 13, dans lequel les horodatages d'identification comprennent un horodatage d'identification de patient (T(pld)) et un horodatage d'identification de dispositif (T(dld)), l'horodatage d'identification de patient étant indicatif d'un horodatage d'identification de patient auquel l'élément de donnée d'identification de patient (pld) a été obtenu, et l'horodatage d'identification de dispositif étant indicatif d'un temps d'identification de dispositif auquel l'élément de données d'identification de dispositif (dld) a été obtenu ; et dans lequel l'identification d'ensembles associés d'événements d'identification et d'événements d'acquisition de données comprend l'identification de triplets d'horodatages d'identification de patient, d'horodatages d'identification de dispositif et d'horodatages d'acquisition de données (T(acq)) respectifs.

15. Procédé selon la revendication 13, dans lequel les horodatages d'identification comprennent un horodatage d'identification de patient (T(pld)) indicatif d'un temps d'identification de patient auquel l'élément de données d'identification de patient (pld) a été obtenu ; et
dans lequel l'identification d'ensembles associés d'événements d'identification et d'événements d'acquisition de données comprend l'identification de duplets d'horodatages d'identification de patient et d'horodatages d'acquisition de données (T(acq)) respectifs.

16. Procédé selon l'une quelconque des revendications précédentes, dans lequel l'ensemble d'éléments de données d'identification comprend en outre un élément de données d'identification d'opérateur (old) identifiant un opérateur du dispositif électronique.

17. Procédé selon l'une quelconque des revendications précédentes, dans lequel la réception d'un horodatage d'acquisition (T(acq)) du dispositif électronique (103) via la deuxième interface de communication comprend en outre la réception, à partir du dispositif électronique, d'un horodatage de commande (TED(trans)) indicatif d'un temps actuel déterminé par le dispositif électronique ; et la correction (S312) de l'horodatage d'acquisition sur la base d'une comparaison de l'horodatage de commande avec un temps actuel déterminé par le système de traitement de données.

18. Système configuré pour réaliser le procédé d'enregistrement de données relatives au patient selon la revendication 1, le système comprenant le dispositif d'enregistrement (102), le dispositif électronique (103) et le système (101) de traitement de données, dans lequel le dispositif d'enregistrement comprend des moyens d'entrée pour recevoir l'ensemble d'identifiants et la première interface de communication adaptée pour transmettre l'élément de données d'identification indicatif de l'identifiant reçu au système de traitement de données ;
dans lequel le dispositif électronique comprend des moyens d'acquisition des données relatives au patient et la deuxième interface de communication adaptée pour communiquer les données relatives au patient acquises au système de traitement de données.

19. Produit-programme d'ordinateur comprenant des moyens de code de programme adaptés pour amener le système selon la revendication 18 à réaliser le procédé selon la revendication 1, lorsque lesdits moyens de code programme sont mis en œuvre par le système.
